# EUROPÄISCHE PATENTSCHRIFT

(11) **EP 0 696 574 B1**
(45) Veröffentlichungstag und Bekanntmachung des Hinweises auf die Patenterteilung: **06.10.1999**
(21) Anmeldenummer: 95111998.1
(22) Anmeldetag: 31.07.1995
(51) Int. Cl.: C07C 209/36, C07C 211/44

(54) **Verfahren zur Herstellung von aromatischen Aminen**
Process for the preparation of aromatic amines
Procédé pour la préparation d'amines aromatiques

(30) Priorität: 08.08.1994 DE 4428018
(43) Veröffentlichungstag der Anmeldung: 14.02.1996
(73) Patentinhaber: BAYER AG, 51368 Leverkusen (DE)
(72) Erfinder: Langer, Reinhard, Dr., D-47800 Krefeld (DE); Buysch, Hans-Josef, Dr., D-47809 Krefeld (DE); Pentling, Ursula, Dr., D-47906 Kempen (DE); Wagner, Paul, Dr., D-40597 Düsseldorf (DE)

(56) Entgegenhaltungen:
- EP-A- 0 011 090
- DE-A- 1 809 711
- GB-A- 1 452 466
- GB-A- 2 182 330
- CHEMICAL ABSTRACTS, vol. 112, no. 4, 22.Januar 1990 Columbus, Ohio, US; abstract no. 26341t, Seite 362; Spalte 2; & CS-A-258 375 (PAVLAS, PAVEL ET AL.) 14.April 1989
- CHEMICAL ABSTRACTS, vol. 100, no. 16, 16.April 1984 Columbus, Ohio, US; abstract no. 123155v, GRAMATIKOV, K. ET AL. 'Adiabatic process of catalytic reduction of nitrobenzene to aniline.' Seite 108; Spalte 2; & IZV. KHIM., Bd. 16, Nr. 1-2, 1983 Seiten 38-43,
- CHEMICAL ABSTRACTS, vol. 117, no. 20, 16.November 1992 Columbus, Ohio, US; abstract no. 194013j, Seite 120; Spalte 2; & CS-A-263 430 (ROZINEK, RADOVAN ET AL.) 20.Januar 1992
- ULLMANN: "Encyclopedia of Industrial Chemistry", VCH VERLAGSGESELLSCHAFT, BASEL

## Beschreibung

Gegenstand der Erfindung ist ein Verfahren zur Hydrierung von Nitroaromaten zu aromatischen Aminen in der Gasphase an ortsfesten Katalysatoren, worin dem Katalysator von außen weder Wärme zugeführt noch Wärme entzogen wird, d.h., daß das Verfahren adiabat durchgeführt wird.

Aromatische Amine sind wichtige Zwischenprodukte, welche preiswert und in großen Mengen zur Verfügung stehen müssen. Daher müssen z.B. für die Hydrierung von Nitrobenzol Anlagen mit sehr großen Kapazitäten gebaut werden.

Die Hydrierung von Nitroaromaten ist eine stark exotherme Reaktion. So werden bei 200°C bei der Hydrierung von Nitroxylol zu Xylidin ca. 488 kJ/mol (117 kcal/mol) und bei der Hydrierung von Nitrobenzol ca. 544 kJ/mol (130 kcal/mol) freigesetzt.

Die Abführung und Verwendung der Reaktionswärme ist demnach ein wichtiger Punkt bei der Durchführung von Verfahren zur Hydrierung von Nitroaromaten.

So wird in einer etablierten Verfahrensweise der Katalysator als fluidisiertes, thermostabilisiertes Bett betrieben (US 3 136 818). Der effektiven Wärmeabfuhr dieser Verfahrensweise stehen Probleme durch uneinheitliche Verweilzeitverteilung (Nitrobenzoldurchbruch) und Katalysatorabrieb gegenüber.

Enge Verweilzeitverteilungen und geringer Katalysatorabrieb sind in Reaktoren mit stationärer Katalysatorschüttung möglich. Jedoch ergeben sich in solchen Reaktoren Probleme mit der Thermostatisierung der Katalysatorbetten. Im allgemeinen werden thermostatisierte Rohrbündelreaktoren verwendet, welche, besonders bei großen Reaktoren, einen sehr aufwendigen Kühlkreislauf besitzen (DE-OS 2 201 528, DE-OS 3 414 714). Derartige Reaktoren sind komplex und verursachen hohe Investitionskosten. Mit der Baugröße rasch ansteigende Probleme bezüglich mechanischer Festigkeit und gleichmäßiger Thermostatisierung der Katalysatorschüttung machen große Aggregate solchen Typs unwirtschaftlich.

Einfache Reaktoren, wie sie für das weiter unten beschriebene erfindungsgemässe Verfahren verwendet werden, enthalten nur Katalysatorschüttungen und besitzen kein System zur Wärmehaushaltung im Reaktor. Sie sind leicht in einen technischen Maßstab zu übertragen, in allen Größen preiswert und robust. Die Reaktionsenthalpie spiegelt sich bei diesem Reaktortyp in der Temperaturdifferenz zwischen Edukt-und Produktgasstrom quantitativ wieder.

Für die stark exotherme Hydrierung von Nitroaromaten wird bisher weder die Verwendung solcher Reaktoren beschrieben, noch werden geeignete Katalysatoren und geeignete Betriebsweisen aufgezeigt.

GB 1.452.466 befasst sich mit einem Verfahren zur Nitrobenzolhydrierung, bei dem ein adiabater Reaktor einem isothermen Reaktor nachgeschaltet ist. Dabei wird der größte Teil des Nitrobenzols in einem thermostatisierten Rohrbündelreaktor umgesetzt, lediglich die Hydrierung des Restgehaltes an Nitrobenzol erfolgt bei relativ geringem Wasserstoffüberschuß (kleiner 30:1) in einem adiabaten Reaktor.

Der Vorteil des völligen Verzichts auf einen thermostatisierten Reaktor bei rein adiabater Umsetzung wurde nicht erkannt.

DE-AS 1 809 711 befaßt sich mit dem gleichmäßigen Einbringen von flüssigen Nitroverbindungen in einen heißen Gasstrom durch Verdüsen, bevorzugt an verengten Stellen unmittelbar vor dem Reaktor. Auf den Aufbau des Reaktors wird in der Anmeldungsschrift nicht eingegangen. Dem Beispiel kann man jedoch entnehmen, daß trotz eines beachtlichen Wasserstoffüberschusses mindestens 34 % der Reaktionsenthalpie den Reaktor nicht mit dem Produktgas verlassen hat.

In DE-OS 36 36 984 wird ein Verfahren zur gekoppelten Produktion von Nitro-und Dinitroaromaten aus den entsprechenden Kohlenwasserstoffen durch Nitrierung und deren nachfolgende Hydrierung beschrieben. Die Hydrierung erfolgt in der Gasphase bei Temperaturen zwischen 176 und 343,5°C. Das Schwergewicht dieser DE-OS liegt in der Ausnutzung der Schmelzpunkterniedrigung von Mono-/Di-nitrobenzol-Mischungen und von Amino-/Diamino-benzol-Mischungen. Dadurch kann sowohl das Produkt der Nitrierung als auch das der Hydrierung ohne Kristallisationsprobleme auch in Substanz, also ohne Lösungsmittel gehandhabt werden. Der Gedanke der adiabaten Reaktionsführung ist dieser DE-OS nicht zu entnehmen.

Daß neue, verbesserte Anlagen zur Nitrobenzolhydrierung zu Anilin in Betrieb genommen wurden, kann man der Zeitschrift "Hydrocarbon Processing 59" (Bd. 59, 1979, Heft 11, S. 136) entnehmen. Der Veröffentlichung ist zu entnehmen, daß die Dampfgewinnung und die Reaktion dabei in einem Verfahrensschritt eng gekoppelt durchgeführt werden.

In allen oben genannten Veröffentlichungen werden Cu-Katalysatoren eingesetzt, die ausschließlich mit niedrigen Belastungen (<0,1 g/ml h) und auf niedrigem Temperaturniveau betrieben werden. Daraus resultieren geringe Raumzeitausbeuten.

Neben den erwähnten Kupferkatalysatoren sind zahlreiche andere Kontakte für die Gasphasenhydrierung von Nitroaromaten beschrieben. Sie sind in vielen Publikationen beschrieben und umfassen als hydrieraktive Elemente und Verbindungen Pd, Pt, Ru, Fe, Co, Ni, Mn, Re, Cr, Mo, V, Pb, Ti, Sn, Dy, Zn, Cd, Ba, Cu, Ag, Au, z.T. als Oxide, Sulfide oder Selenide und auch in Form einer Raney-Legierung, auf Trägern, wie Al₂O₃, Fe₂O₃/Al₂O₃, SiO₂, Silikate, Kohle, TiO₂, Cr₂O₃.

Auch diese Katalysatoren werden mit nur geringen Belastungen in einem Temperaturbereich unterhalb 350°C betrieben.

Die Erfindung betrifft ein Verfahren zur Herstellung von aromatischen Aminen der Formel 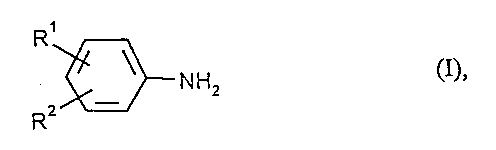 in der
- R¹ und R,: unabhängig voneinander Wasserstoff, Methyl oder Ethyl bedeuten, wobei
- R¹: zusätzlich Amino bedeuten kann,
durch Hydrierung von Nitroaromaten der Formel 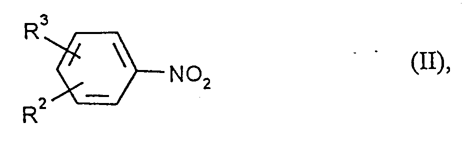 in der
- R² und R³: unabhängig voneinander Wasserstoff, Methyl oder Ethyl bedeuten, wobei R³ zusätzlich Nitro bedeuten kann,
mit H₂ an ortsfesten Katalysatoren, dadurch gekennzeichnet ist, daß bei einem Druck von 1 bis 30 bar, einer Eingangstemperatur des Nitroaromat/H₂-Gemisches von 200 bis 400°C, einem Verhältnis von zwischen 60 und 800 Äquivalenten Wasserstoff pro Nitrogruppenäquivalent nach Homogenisierung der Gasmischung und einer maximalen Katalysatortemperatur von 500°C unter adiabatischen Bedingungen gearbeitet wird. Bevorzugte Nitroaromaten für das erfindungsgemäße Verfahren sind solche der Formel 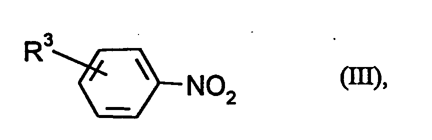 in denen
- R³: die obige Bedeutung hat.

Besonders bevorzugt als Nitroaromat ist Nitrobenzol.

Eine erfindungsgemäße Produktionsanlage besteht aus mindestens einem adiabaten Reaktor mit stationärem Katalysator. Es werden maximal 10, bevorzugt maximal 5, besonders bevorzugt maximal 3 solcher Reaktoren hintereinander geschaltet. Jeder in Reihe geschaltetete Reaktor kann durch mehrere parallel geschaltete ersetzt werden. Es werden maximal 5, bevorzugt maximal 3, besonders bevorzugt maximal 2 Reaktoren im Ersatz des einen Reaktors parallel geschaltet. Das erfindungsgemäße Verfahren kann demnach maximal 50 und minimal einen Reaktor beinhalten.

Mehrere Reaktoren mit einer Katalysatorschüttung können durch weniger Reaktoren mit mehreren Katalysatorschüttungen ersetzt werden.

Die Reaktoren bestehen aus einfachen Behältern mit isolierten Katalysatorschüttungen, wie sie z.B. in Ullmanns Encyclopedia of Industrial Chemistry (Fifth, Completely Revised Edition, Vol. B4, Seite 95-102, Seite 210-216) beschrieben werden.

Die Katalysatorschüttungen werden nach dem Stand der Technik auf oder zwischen gasdurchlässigen Wandungen angebracht. Insbesondere bei dünnen Schüttungen werden oberhalb, unterhalb oder oberhalb und unterhalb der Schüttung technische Vorrichtungen zur gleichmäßigen Gasverteilung angebracht. Dies können Lochplatten, Glockenböden, Ventilböden oder andere Einbauten sein, die durch Erzeugung eines geringen, aber gleichmäßigen Druckverlusts einen gleichförmigen Eintritt des Gases in die Katalysatorschüttung bewirken, bevorzugt werden Metall-oder Keramiksintermatten benutzt, wie sie z.B. die Firma Krebsöge vertreibt.

Statt Katalysatorschüttungen können auch als Trägermaterial geeignete Packungen verwendet werden. Dies wären z.B. Wabenkörper oder gewellte Schichten, wie sie von der Firma Sulzer unter dem Namen Katapak angeboten werden. Diese Packungen werden zum erfindungsgemäßen Einsatz vor dem Einbringen in den Reaktor durch das Aufbringen geeigneter Metallverbindungen aktiv gemacht.

Vor jeder Katalysatorschüttung wird frischer Nitroaromat in den Kreisgasstrom, der hauptsächlich aus recyclisiertem und frisch zugesetztem Wasserstoff besteht, eindosiert. Das kann so geschehen, wie in DE 1 809 711 beschrieben, bevorzugt wird jedoch der Nitroaromat im Frischwasserstoff vollständig verdampft und dann gasförmig in den Kreisgasstrom gegeben. Der Vorteil dieser Verfahrensweise liegt in der deutlich geringeren Bildung von Ablagerungen im Reaktor und in den Zuleitungen. Die Verdampfung kann nach dem Stand der Technik in bekannten Verdampfern erfolgen, wie z.B. Fallfilm-, Steigrohr-, Einspritz-, Dünnschicht-, Umlauf- und Wendelrohrverdampfern. Bevorzugt wird in Fallfilm- und Einspritz-, ganz besonders bevorzugt in Fallfilmverdampfern verdampft. Weiterhin ist die Verdüsung des flüssigen Nitroaromaten in den Frischwasserstoff- oder Kreisgaswasserstoffstrom mittels Einstoff- oder Zweistoffdüsen möglich, wobei die Vereinigung des Eduktgasstromes nach einer Überhitzung in einem Wärmetauscher erfolgen kann. Der Verdampfung kann eine grundsätzlich bekannte Tröpfchenabscheidung nachgeschaltet werden. Der Eduktgasstrom wird in bekannter Weise mittels entsprechender Zuführung und Verteilung und oder durch Vermischungseinrichtungen, wie z.B. Mischer vom Typ SUX oder SMV der Firma Sulzer oder Kenics im Kreisstrom vermischt.

Nach jeder Katalysatorschüttung wird das sie verlassende Produktgas unter Dampfgewinnung abgekühlt. Dazu leitet man es durch einen oder mehrere Wärmetauscher. Dies können die dem Fachmann bekannten Wärmetauscher, wie z.B. Rohrbündel-, Platten-, Ringnut-, Spiralstrom-, Rippenrohrwärmetauscher sein.

Nach Verlassen des letzten Wärmetauschers zur Dampferzeugung wird das Produktgas abgekühlt, um aromatisches Amin und Reaktionswasser aus dem Reaktionsgemisch zu entfernen. Danach wird das verbleibende Kreisgas, nach Abzweigen einer geringen Gasmenge zum Konstanthalten von gasförmigen Komponenten des Kreisgases, die z.T. mit den Edukten, hauptsächlich dem Frischwasserstoff eingeschleppt werden und z.T. auf dem Kontakt entstehen (N₂, NH₃), wieder dem ersten Reaktor zugeführt. Vorher muß es auf die Eingangstemperatur vorgewärmt werden und frisches Edukt zugemischt erhalten.

Vorteilhaft verwirklicht man das Abkühlen des Produktgases und das Aufwärmen des Kreisgases - nach dem Ausschleusen der kondensierbaren Produkte und dem Abzweigen des Kreisgasaustrages - indem man die Gasströme im Gegenstrom durch Wärmetauscher aneinander vorbeiführt.

Das Kreisgas wird unmittelbar vor dem ersten Reaktor mittels eines Wärmetauschers wieder auf Eingangstemperatur gebracht. Davor oder danach, bevorzugt danach, wird Nitroaromat und Frischwasserstoff wie oben beschrieben eindosiert.

Das Austragen der Produktkomponenten aus dem abgekühlten Kreisgasstrom kann mittels Partial- und/oder Totalkondensation oder durch Herauswaschen mit kaltem Produkt oder mit einem inerten Lösungsmittel erfolgen, bevorzugt wird der Gasstrom zuerst durch einen Wäscher geführt, in dem ihm das Kondensat eines nachgeschalteten gekühlten Kondensators entgegenfließt. Der Wäscher wird so betrieben, daß das ihn verlassende Kondensat einphasig ist. Hierzu muß das ihn verlassende Kondensat in Abhängigkeit vom Druck und von der Art des aromatischen Amins eine bestimmte Temperatur haben, um kein oder wenig Wasser im Kondensat zu haben. Die Optimierung dieses Problems ist dem Fachmann geläufig. Dem Kondensator zum Betrieb des Wäschers ist in der bevorzugten Ausführung ein zweiter Kondensator nachgeschaltet, in dem der Hauptteil des Wassers und ein weiterer Teil des aromatischen Amins niedergeschlagen wird. Dieses Kondensat wird einer Vorrichtung zur Trennung flüssiger Phasen (organisch und wäßrig) zugeleitet. Die organische Phase wird mit dem Wäschekondensat einer destillativen Aufarbeitung zugeführt, die wäßrige Phase wird ebenfalls einer Aufarbeitung unterworfen, um gelöstes aromatisches Amin abzutrennen und der destillativen Aufarbeitung zuzuführen.

Das Kreisgas hat nach dem Verlassen des letzten Kondensators in Abhängigkeit vom Absolutdruck eine Temperatur zwischen 5 und 95°C, bevorzugt zwischen 10 und 80°C, besonders bevorzugt zwischen 15 und 70°C, ganz besonders bevorzugt zwischen 20 und 60°C.

Von diesem Gas wird eine geringe Menge zum Ausschleusen der oben genannten gasförmigen Bestandteile abgetrennt.

Danach wird das Kreisgas einem Kompressor zugeführt, durchläuft die Gegenstromwärmetauscher und gegebenenfalls einen Überhitzer, um in den ersten Reaktor geleitet zu werden. Der Kompressor kann prinzipiell an jeder Stelle des Gaskreislaufs stehen, bevorzugt steht er an Stellen, in denen nicht mit Ablagerungen zu rechnen ist, d.h. nach der Kondensation, vor dem 1. Reaktor und auf niedrigem Temperaturniveau.

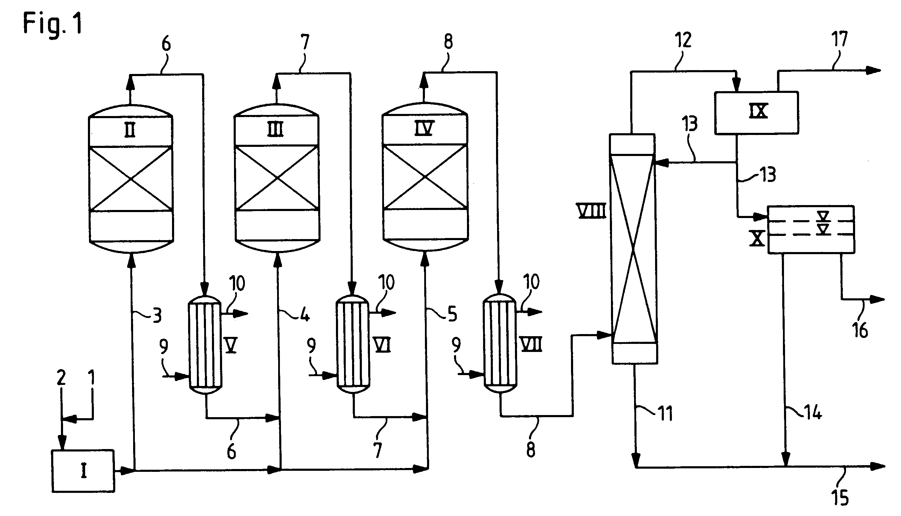

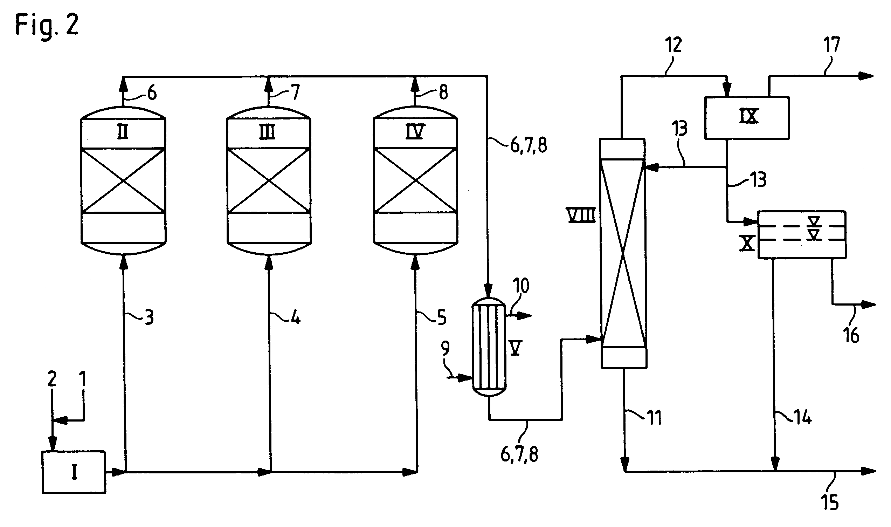

Erfindungsgemäße Produktionsanlagen sind in Figur 1 und 2 mit Mengenströmungen, Drücken und Temperaturen beispielhaft aufgeführt. Die Angaben sollen nicht einschränkend wirken.

Das erfindungsgemäße Verfahren wird bei Drücken zwischen 1 und 30, bevorzugt zwischen 1 und 20, besonders bevorzugt zwischen 1 und 15 bar betrieben.

Vor jedem Reaktor wird Frischwasserstoff und Nitroaromat in den Kreisgasstrom dosiert. Pro Nitrogruppenäquivalent sind nach der Homogenisierung der entstehenden Gasmischung zwischen 60 und 800, bevorzugt zwischen 80 und 400, besonders bevorzugt zwischen 100 und 300, ganz besonders bevorzugt zwischen 120 und 200 Äquivalente Wasserstoff zugegen.

Die Temperatur des homogenisierten Eduktgasgemisches an jedem Reaktoreingang liegt zwischen 200 und 400, bevorzugt zwischen 230 und 370, besonders bevorzugt zwischen 250 und 350°C.

Die Dicke der Katalysatorschüttungen kann zwischen 1 cm und 5 m, bevorzugt zwischen 5 cm und 2 m, besonders bevorzugt zwischen 10 cm und 1 m, ganz besonders bevorzugt zwischen 30 cm und 60 cm liegen.

Als Katalysatoren können alle bisher für die Gasphasenhydrierung von Nitroverbindungen beschriebenen Kontakte eingesetzt werden. Diese enthalten die weiter oben genannten Elemente, entweder als Legierung oder als Mischoxide und gegebenenfalls auf inertem Trägermaterial. Als Trägermaterialien kommen besonders in Frage: α-und γ-Al₂O₃, SiO₂, TiO₂, Roterde und Limonit, Fe₂O₃/Al₂O₃-Mischungen, CuO/Cr₂O₃-Mischungen, Wasserglas , Graphit, Aktivkohle (BET 20-100 m²/g) und Kohlefasern. Es können aber prinzipiell auch andere Träger eingesetzt werden.

Bevorzugt werden Katalysatoren aus DE-OS 2 849 002 eingesetzt. Dies sind Trägerkatalysatoren auf inerten Trägern mit einer BET-Oberfläche von weniger als 20 m²/g, bzw. α-Al₂O₃ mit einer BET von weniger als 10 m²/g. Die in DE-OS 2 849 002 beschriebene Vorbehandlung mit einer Base ist nicht unbedingt erforderlich.

Auf dem Trägermaterial sind drei Aktivstoffklassen niedergeschlagen:
(a) 1-100 g/l Katalysator eines oder mehrer Metalle der Gruppen VIIIa, Ib und IIb des Periodensystems der Elemente (Mendeljew),
(b) 1-100 g/l eines oder mehrerer Übergangsmetalle der Gruppen IIb, IVa, Va und VIa und
(c) 1-100 g/l eines oder mehrerer Hauptgruppenelemente der Gruppen IVb und Vb.

Elemente der Gruppe IIb können somit als Aktivstoffe (a) und (b) wirken. Bevorzugte Aktivstoffe sind Pd als Metall (a), V, Nb, Ta, Cr, Mo, W, Ti als Übergangsmetall (b) und Pb und Bi als Hauptgruppenelemente (c).

Besonders bevorzugt werden
(a) 20-60 g Pd,
(b) 20-60 g V und
(c) 10-40 g Pb auf den Träger gebracht.

Die Aktivstoffe werden in Form ihrer löslichen Salze auf den Träger gebracht, gegebenenfalls sind mehrere Behandlungen (Tränkungen) pro Komponente erforderlich. In bevorzugter Weise werden die Aktivstoffe nur schalenförmig, d.h. in der Nähe der Oberfläche des Katalysators aufgebracht.

Diese Kontakte werden in einem Temperaturbereich betrieben, der zwischen der Eingangstemperatur des Eduktgases und max. 500, bevorzugt maximal 480, besonders bevorzugt maximal 460, ganz besonders bevorzugt maximal 440°C liegt.

Weitere bevorzugte Katalysatoren sind solche, die Pd alleine oder mit Rh und/oder Ir und/oder Ru auf Kohleträgern mit geringer BET-Oberfläche tragen. Solche Trägermaterialien sind graphithaltig, es sind Graphite selbst und Kokse, wie Nadelkoks oder Petrolskoks. Diese Träger haben eine BET-Oberfläche von 0,2-10 m²/g. Verwendung finden erfindungsgemäß Katalysatoren, die auf Graphit oder graphithaltigem Koks als Träger 0,001-1,5 Gew.-% Pd, bezogen auf das Gesamtgewicht des Katalysators, enthalten, wobei das Pd zu 0 - 40 Relativprozent seiner Menge durch Ir und/oder Rh und/oder Ru ersetzt sein kann. Diese Katalysatoren enthalten also das (die) Edelmetall(e) in folgenden Anordnungen auf dem Träger: Pd allein, Pd/Ir, Pd/Rh, Pd/Ru, Pd/Ir/Rh, Pd/Ir/Ru, Pd/Rh/Ru, Pd/Ir/Rh/Ru. In vielen Fällen werden eine der genannten Zweierkombinationen oder Pd allein eingesetzt. In bevorzugter Weise liegt in den Katalysatoren auf Kohleträgern das Palladium in einer Menge von 0,005-1 Gew.-%, bevorzugt 0,05-0,5 Gew.-%, bezogen auf das Gesamtgewicht des Katalyators, vor. Die Untergrenze Null der Relativprozente der anderen genannten Platinmetalle zeigt den Gebrauch von Pd alleine an. Falls die anderen Platinmetalle eingesetzt werden, beträgt ihr Anteil bevorzugt insgesamt 10-40 Relativprozent; untereinander beträgt ihr Gewichtsverhältnis 1:1-3:1 zwischen je zweien.

Es hat sich weiterhin als vorteilhaft erwiesen, die genannten Katalysatoren zusätzlich mit einer schwefelhaltigen oder phosphorhaltigen, bevorzugt phosphorhaltigen Verbindungen zu dotieren. Ein solcher zusätzlicher Gehalt an Dotierungsmittel beträgt 0,1-2 Gew.-%, bevorzugt 0,1-1 Gew.-% Schwefel oder Phosphor, bevorzugt Phosphor, in chemisch gebundener Form, bezogen auf das Gesamtgewicht des Katalysators. Als phosphorhaltige Verbindungen für die Dotierung der erfindungsgemäßen Katalysatoren sind bevorzugt zu nennen: die Sauerstoffsäuren des Phosphors H₃PO₄, H₃PO₃, H₃PO₂ oder deren Alkalisalze, wie z.B. Natriumdihydrogenphosphat, Natrium- oder Kaliumphosphat oder Natriumhypophosphit.

Zur Herstellung der Katalysatoren auf Kohleträgern kann so vorgegangen werden, daß auf einen der genannten Träger in Form von Pillen, Kugeln, Stranggranulat oder Bruchsrücken von etwa 1-10 mm Abmessung die genannten Edelmetalle in Form geeigneter Salze sowie die schwefelhaltige oder phosphorhaltige Verbindung in getrennten Arbeitsgängen aufgetragen werden, wobei nach jedem Auftrag getrocknet wird. Das Trocknen geschieht in bekannter Weise, beispielsweise bei 100-140°C und vermindertem bis normalem Druck, beispielsweise bei 1-1.000 mbar; als verminderter Druck kommt beispielsweise der einer Wasserstrahlpumpe in Betracht. Zum Tränken des Trägers können wäßrige Lösungen verwendet werden. Dies ist in bevorzugter Weise bei den schwefel- oder phosphorhaltigen Verbindungen, von denen wasserlösliche bevorzugt werden, der Fall. Die Edelmetallsalze werden jedoch bevorzugt in organischen Lösungsmitteln, wie einfachen Alkoholen, Ketonen, cyclischen Ethern oder Nitrilen, gelöst und aufgetragen. Beispiele für solche organischen Lösungsmittel sind Methanol, Ethanol, Propanol, Isopropanol, Aceton, Methyl-ethylketon, Dioxan, Acetonitril und vergleichbare Lösungsmittel. Bei Salzen mit organischen Anionen können auch Methylenchlorid und vergleichbare Lösungsmittel eingesetzt werden. Geeignete Salze der Edelmetalle sind beispielsweise ihre Chloride, Nitrate oder Acetate.

Nach dem Imprägnieren und dem abschließenden Trocknen steht der Katalysator zur Verfügung. Er wird in bevorzugter Weise im Reaktor vor Beginn der Hydrierung von Nitrolbenzol durch eine Behandlung mit Wasserstoff bei erhöhter Temperatur aktiviert. Eine solche erhöhte Temperatur liegt beispielsweise im Bereich von 200-400°C, bevorzugt im Bereich von 200-380°C.

Die genannten Katalysatoren sind in hervorragender Weise zur Hydrierung von Nitrobenzol zu Anilin verwendbar.

Bei Aktivitätsabfall des eingesetzten Katalysators kann er leicht in situ, d.h. im Hydrierreaktor, regeneriert werden. Hierzu wird der Katalysator bei 350-400°C nacheinander mit Wasserdampf, einem Stickstoff/Luft-Gemisch oder atmosphärischer Luft und schließlich Stickstoff behandelt. Die Behandlung mit Wasserdampf kann 1 bis 3 Stunden, die Behandlung mit Luft bzw. dem Stickstoff/Luft-Gemisch kann 1 bis 4 Stunden erfolgen. Eine solche Regenerierung ist nicht möglich bei Edelmetallkatalysatoren auf anderen als den beschriebenen Kohleträgern, z.B. bei aktivierter Kohle als Träger, da eine aktivierte Kohle bei einer solchen Regenerierung zu verbrennen beginnt. Zur erneuten Aktivierung des Katalysators kann sich eine Behandlung mit Wasserstoff bei 200- 400°C anschließen.

Diese Katalysatoren werden im Temperaturbereich unter 480°C, bevorzugt unter 460°C, ganz besonders bevorzugt unter 440°C betrieben.

Die als bevorzugt beschriebenen Kontakte ermöglichen eine besonders lange Laufzeit zwischen den Regenerierungen.

Prinzipiell können die Katalysatorkörner jede beliebige Form aufweisen, wie z.B. Kugeln, Stäbchen, Raschigringe oder Granulat oder Tabletten. Bevorzugt werden Formkörper benutzt, deren Schüttungen einen niedrigen Stromungswiderstand bei guten Gas-Oberflächen-Kontakt aufweisen, wie z.B. Raschigringe, Sattelkörper, Wagenräder und Spiralen.

Das erfindungsgemäße Verfahren erlaubt besonders vorteilhafte Durchführungen der Gasphasenhydrierung, die zu konstant hohen Selektivitäten an aromatischem Amin und langen Katalysatorstandzeiten zwischen den Regenerationen des Katalysators führen, die i.a. im Abbrennen von kohlenstoffreichen Ablagerungen bestehen.

Eine Vorgehensweise besteht darin, daß bei konstantem Druck gearbeitet wird, und daß der Katalysator mit besonders hoher Belastung angefahren wird. Die Belastung wird dann im Zuge der Katalysatordesaktivierung während der Laufzeit zwischen zwei Regenerierungen so zurückgenommen, daß kein Nitroaromat durchbricht.

Eine andere gleichwertige Methode besteht darin, die Belastung des Katalysators konstant zu halten und mit niedrigem Systemdruck zu beginnen; kurz bevor Nitroaromat durchzubrechen beginnt, wird der Systemdruck langsam gesteigert.

Es kann auch eine Fahrweise zwischen den beiden Extremen konstanter Druck und konstante Belastung gewählt werden. Bevorzugt wird mit niedrigem Druck und niedriger Belastung angefahren, um dann im Verlauf der Katalysatordesaktivierung beide Größen zu steigern.

Die Belastung der Katalysatoren kann beim erfindungsgemäßen Verfahren sehr hoch sein und 0,1 g bis zu 20 g Nitroaromat pro ml Kat. und Stunde, bevorzugt bis zu 15 g/ml·h, besonders bevorzugt bis zu 10 g/ml·h, ganz besonders bevorzugt bis zu 5 g/ml·h betragen.

Das erfindungsgemäße Verfahren zeichnet sich demnach durch hohe Raumzeitausbeuten aus, verbunden mit einer Verringerung der Apparategrößen. Das erfindungsgemäße Verfahren erlaubt ferner einfache Standard-Apparaturen zu benutzen und ermöglicht hohe Anlagenkapazitäten bei geringen Investionskosten. Das Verfahren ist besonders geeignet, um Nitrobenzol zu Anilin umzusetzen.

Die beigefügten Fig. 1 und Fig. 2 zeigen Beispiele für drei hintereinander geschaltete (Fig. 1) bzw. drei parallel geschaltete Reaktoren und erläutern ferner die Beispiele 9 und 10. Als Apparate sind dargestellt: I = Verdampfer; II, III, IV = drei Reaktoren; V, VI, VII = drei Dampferzeuger (in Fig. 2 nur einer); VIII = Destillationskolonne; IX = Kondensator; X = Trenngefäß mit zwei Niveauhaltungen. Als Stoffströme sind dargestellt: 1 = H₂; 2 = Nitrobenzol als Beispiel für einen Nitroaromaten; 3, 4, 5 = Einspeisungen für H₂/Nitrobenzol-Dampfgemisch in die Reaktoren; 6, 7, 8 = hydriertes Reaktionsgemisch aus den Reaktoren, das in Fig. 1 nach Abkühlung zur Nutzdampf-Erzeugung in den jeweils folgenden Reaktor unter Zuspeisung von frischem H₂/Nitrobenzol-Dampfgemisch geleitet wird und in Fig. 2 gesammelt und gemeinsam der Dampferzeugung zugeführt wird; 9 = Kesselspeisewasser; 10 = Nutzdampf; 11 = H₂O-haltiges Anilin aus dem Sumpf von VIII; 12 = H₂O/Anilin-Dampfgemisch; 13 = Rücklauf auf VIII und Zulauf zu X; 14 = H₂O-haltiges Anilin aus X; 15 = Sammelleitung für H₂O-haltiges Anilin zur weiteren Aufarbeitung; 16 = Anilin-haltiges Wasser aus X zur weiteren Aufarbeitung; 17 = Abgas, das wegen seines Gehalts an H₂ größtenteils in den Prozeß zurückgeführt wird (nicht gezeichnet) und zum kleineren Teil entsorgt wird (z.B. durch Verbrennung).

### Beispiel 1

4000 g Graphitgranulat EG 17 der Firma Ringsdorf, Granulat 1-3 mm, Rüttelgewicht 650-1000 g/l, BET = 0,3-0,4 m²/g) mit einer Saugfähigkeit von 7 ml Acetonitril pro 100 g Träger wurden in einem drehbaren Gefäß vorgelegt und mit einer Lösung aus 16,6 g Palladium-II-acetat in 260 g Acetonitril versetzt. Die Mischung wurde durch Drehen bis zur völligen Aufnahme der Lösung vom Trägermaterial bewegt. Danach erfolgte eine fünfminütige Trocknung des Feststoffes in einem starken aufsteigenden 40°C warmen Luftstrom. Der getrocknete Katalysator wurde anschließend 3 Stunden in einem 100°C heißem Wasserstoffstrom reduziert.

### Beispiel 2

5000 ml α-Al₂O₃ der Firma Condea (α-Tonerde, Dichte 1,02 g/ml,Kugeln mit einem Durchmesser von 1 mm, BET = 4 m²/g) mit einer Saugfähigkeit von 33,4 ml Wasser pro 100 g Träger wurden in einem drehbaren Gefaß vorgelegt und mit einer Lösung aus 553 g Dinatrium-tetra-chloropalladat in 1200 g Wasser versetzt. Die Mischung wurde solange durch Drehen vermischt, bis die gesamte Lösung vom Trägermaterial aufgenommen worden war. Danach erfolgte eine 10minütige Trocknung des Feststoffes in einem starken aufsteigenden 40°C warmen Luftstrom. Der getrocknete Katalysator wurde 3 Stunden bei 350°C mit Wasserstoff reduziert, der reduzierende und getrocknete Feststoff anschließend bei Raumtemperatur mit einer Lösung aus 500 g Oxalsäuredihydrat und 178,6 g Vanadiumpentoxid in 1030 g Wasser versetzt und solange durch Drehen vermischt bis die gesamte Lösung vom Trägermaterial aufgenommen worden war. Danach erfolgte eine 10minütige Trocknung des Feststoffes in einem starken aufsteigenden 40°C warmen Luftstrom, worauf eine erneute Tränkung mit der gleichen Menge einer Vanadiumoxalat-Lösung mit anschließender Trocknung im warmen Luftstrom folgte. Der getrocknete Katalysator wurde 4 Stunden bei 300°C getempert und dann auf Raumtemperatur abgekühlt. Es folgte eine Tränküng des Feststoffes, wie oben beschrieben, mit einer Lösung aus 128,2 g Blei-II-acetat-trihydrat in 1200 g Wasser. Danach wurde der Feststoff wiederum in einem starken aufsteigenden 40°C warmen Luftstrom 10 Minuten getrocknet, der getrocknete Katalysator 3 Stunden bei 350°C mit Wasserstoff reduziert und abschließend bei Raumtemperatur mit destilliertem Wasser gewaschen, bis das Waschwasser einen pH-Wert von 7 aufwies. Der so erhaltene Katalysator erfuhr eine abschließende 10minütige Trocknung in einem stark aufsteigenden Luftstrom bei 40°C.

### Beispiel 3

4000 g Nadelkoks der Firma Grafogran GmbH (Granulat 1-4 mm, BET = 1,0-1,1 m²/g) mit einer Saugfähigkeit von 35 ml Wasser pro 100 g Träger wurden in einem drehbaren Gefäß vorgelegt und mit einer Lösung aus 16,6 g Palladium-II-acetat in 260 g Acetonitril versetzt. Die Mischung wurde solange durch Drehen vermischt, bis die gesamte Lösung vom Trägermaterial aufgenommen worden war. Danach erfolgte eine 5minütige Trocknung des Feststoffes in einem starken aufsteigenden 40°C warmen Luftstrom. Der getrocknete Katalysator wurde anschließend 3 Stunden in einem 100°C heißen Wasserstoffstrom reduziert.

### Beispiel 4

5000 ml α-Al₂O₃ der Firma Condea (α-Tonerde, Dichte 1,02 g/ml, Kugeln mit einem Durchmesser von 1 mm, BET = 4 m²/g) mit einer Saugfähigkeit von 33,4 ml Wasser pro 100 g Träger wurden in einem drehbaren Gefäß vorgelegt und mit einer Lösung aus 415 g Dinatrium-tetra-chloropalladat in 1200 g Wasser versetzt. Die Mischung wurde solange durch Drehen vermischt, bis die gesamte Lösung vom Trägermaterial aufgenommen worden war. Danach erfolgte eine 10minütige Trocknung des Feststoffes in einem starken aufsteigenden 40°C warmen Luftstrom, worauf der Tränkungs-Trocknungs-Vorgang wiederholt wurde. Der getrocknete Katalysator wurde 3 Stunden bei 350°C mit Wasserstoff reduziert, der reduzierte und getrocknete Feststoff anschließend bei Raumtemperatur mit einer Lösung aus 500 g Oxalsäsuredihydrat und 178,2 g Vanadiumpentoxid in 1030 g Wasser versetzt und solange durch Drehen vermischt, bis die gesamte Lösung vom Trägermateiral aufgenommen worden Mar. Danach wurde der Feststoff in einem starken aufsteigenden 40°C warmen Luftstrom 10 Minuten getrocknet, worauf zwei neuerliche Tränkungen mit einer Vanadiumoxalat-Lösung mit anschließender Trocknung im warmen Luftstrom folgten. Der getrocknete Katalysator wurde 4 Stunden bei 300°C getempert und dann auf Raumtemperatur abgekühlt. Es folgte eine Tränkung des Feststoffes mit einer Lösung aus 192,4 g Blei-II-acetat-trihydrat in 1200 g Wasser, wie in Beispiel 2 beschrieben. Danach wurde der Feststoff wiederum in einem starken aufsteigenden 40°C warmen Luftstrom 10 Minuten getrocknet, der getrocknete Katalysator 3 Stunden bei 350°C mit Wasserstoff reduziert und abschließend bei Raumtemperatur mit destilliertem Wasser gewaschen, bis das Waschwasser einen pH-Wert von 7 aufwies. Der so erhaltene Katalysator erfuhr eine abschließende 10minütige Trocknung in einem starken aufsteigenden Luftstrom bei 40°C.

### Beispiel 5

3000 ml (2860 g) des in Beispiel 1 hergestellten Katalysators wurden als 155 mm hohe Schüttung in die Mitte eines 1 m langen druckfesten Stahlrohres eingebracht (DIN 2463, ⌀ₐ = 168,3 mm, ⌀ᵢ = 158,3 mm). In der Rohrachse befand sich ein Stahlröhrchen, in dem ein Thermoelement in axialer Richtung verschoben werden konnte. Ober- und unterhalb der Katalysatorschüttung befanden sich Schüttungen aus Glaskugeln. Das druckfeste Stahlrohr wurde mit Gewebeband gut isoliert und in einem mit Schamotte ausgekleideten Rohrofen angeordnet, am oberen Ende mit einem druckfesten Verdampfer-Überhitzer und am unteren Ende mit einem Kondensator mit Auffangsgefäß und kontinuierlicher Produktausschleusung versehen. Durch den Verdampfer-Überhitzer, den Reaktor mit dem Katalysator und den Kondensator mit kontinuierlicher Ausschleusung des Produktes wurde unter Normaldruck ein Wasserstoffstrom geschickt und der Katalysator 3 Stunden bei 100°C aktiviert. Darauf folgend wurde der Druck der Anlage auf 2 bar gesetzt und Nitrobenzol mittels einer Dosierpumpe in den Verdampfer-Überhitzer gefördert.

Der Versuch lief unter folgenden Rahmenbedingungen: pro dosiertes Mol Nitrobenzol wurden 81 Mole Wasserstoff in den Verdampfer-Überhitzer geleitet, die Beheizung des Überhitzers und des Rohrofens wurde so geregelt, daß die Temperatur des Katalysators an keiner Stelle 400°C überschritt, und die Belastung des Katalysators betrug 1 g Nitrobenzol pro ml Schüttung und Stunde. Die gaschromatographische Analyse des Kondensates nach 24 Stunden zeigte, daß das Nitrobenzol zu 99,9 % umgesetzt war und Anilin mit einer Selektivität von mehr als 99,8 % gebildet worden war.

Danach wurden während der nächsten 500 Stunden Druck und Belastung schrittweise auf 4 bar und 3 g/ml·h gesteigert, wobei auch die Katalysatortemperatur oberhalb von 3 bar auf 440°C ansteigen durfte. Die gaschromatographischen Analysen des Kondensates zeigten 100 %igen Umsatz bei einer Selektivität bezüglich Anilin von besser als 99,5 % an. Bei einer Belastung von 3 g/ml·h, einem Nitrobenzol-Wasserstoff-Verhältnis von 1 zu 81, einer maximalen Katalysatortemperatur von 440°C und einem Gesamtdruck von 4 bar wurde der Versuch weitere 500 Stunden ohne Anzeichen einer beginnenden Katalysatordesaktivierung fortgeführt und dann abgebrochen.

### Beispiel 6

2800 ml (3560 g) des in Beispiel 2 hergestellten Katalysators wurden als 160 mm hohe Schüttung in die in Beispiel 5 beschriebene Apparatur eingebracht. Der Katalysator erfuhr anschließend eine 48stündige Konditionierung bei 480°C und 5 bar im Wasserstoffstrom. Dann wurde der Druck auf Normaldruck reduziert und Nitrobenzol und Wasserstoff mit einem molaren Verhältnis von 1 zu 81 in den Verdampfer-Überhitzer gegeben. Die Belastung bezüglich Nitrobenzol wurde auf 1,5 g/ml·h eingeregelt und die Temperatur von Überhitzer und Rohrofen so gewählt, daß der Katalysator an keiner Stelle heißer als 445°C war. Nach 12 Stunden wurde der Druck auf 2 bar heraufgesetzt. Nach weiteren 12 Stunden wurde die Belastung auf 3 g/ml·h und der Gesamtdruck auf 4 bar heraufgesetzt. Gaschromatographische Analysen von aufgefangenem Kondensat zeigten bis zu diesem Zeitpunkt immer 100 % Umsatz bei Selektivitäten bezüglich Anilin von besser als 95 % an.

Nach 100 Stunden wurde der Gesamtdruck auf 8 bar heraufgesetzt. Nach 200 Stunden wurde der Gesamtdruck auf 12 bar heraufgesetzt. Nach 400 Stunden wurde der Gesamtdruck auf 16 bar heraufgesetzt und die Belastung auf 5 g/ml^{·}h erhöht. Nach 600 Stunden wurde der Gesamtdruck auf 20 bar heraufgesetzt. Nach 800 Stunden wurde der Versuch bei 100 %igem Umsatz und einer Selektivität bezuglich Anilin von 99,7 % ohne Anzeichen eienr Desaktivierung des Katalysators abgebrochen. Während des Versuches wanderte die Stelle an der der Katalysator 445°C erreichte vom Katalysatorbettanfang 100 mm in Richtung Katalysatorbettende.

### Beispiel 7

Die Durchführung erfolgte in einer technischen Anlage, wie sie in Fig. 1 dargestellt und weiter oben erläutert wurde. Zu den Stoffströmen in Fig. 1 gehören folgende Mengenangaben (Gewichtsteile pro Stunde = T.) und Temperaturen:
1: 420, 5 T. H₂ + 7,0 T. N₂, 20°C; 2: 8108, 2 T. Nitrobenzol, 20°C; 3. 2383 T. Nitrobenzol + 3441 T. H₂ + 181,5 T. Anilin + 1237,3 T. H₂O + 943,3 T. N₂ (unter Einbeziehung von rückgeführtem Abgas), 220°C; 4:2690 T. Nitrobenzol + 3560,4 T. H₂ + 1989,4 T. Anilin + 1971 T. H₂O + 973 T. N₂ (einschl. Abgasanteil), 220°C; 5:3036 T. Nitrobenzol + 3695 T. H₂ + 4030 T. Anilin + 2799 T. H₂O + 1006 T. N₂ (mit Abgasanteil), 220°C; 6:3324 T. H₂ + 1984 T. Anilin + 1934 T. H₂O + 943,3 T. N₂, 400°C; 7. 3428,3 T. H₂ + 4024 T. Anilin + 2758 T. H₂O + 973 T. N₂, 400°C; 8: 3546 T. H₂ + 6326 T. Anilin + 3687 T. H₂O + 1006 T. N₂, 400°C; 15: 6019 T. Anilin + 97 T. H₂O, 110°C; 16:113,4 T. Anilin + 2267,5 T. H₂O, 60°C; 17:3546 T. H₂ (davon in den Prozeß zurückgeführt 3523,8 T., ausgeschleust zur Entsorgung 22,2 T.) + 194 T. Anilin (192,8 T.; 1,2 T.) + 1323 T. H₂O (1314,7 T.; 8,3 T.) + 1006 N₂ (999 T.; 7 T.), 60°C.

Der Druck lag im Bereich von 5 bis 5,5 bar. Abkühlung nach dem letzten Dampferzeuger auf 180°C.

### Beispiel 8

Die Durchführung erfolgte, wie in Fig. 2 dargestellt und weiter oben erläutert wurde. Zu den Stoffströmen in Fig. 2 gehören folgende Mengenangaben und Temperaturen:
1: 747,4 T. H₂ + 7 T. N₂, 20°C; 2: 8108, 3 T. Nitrobenzol, 20°C; 3, 4 und 5 gemeinsam und aufgeteilt in 3 gleiche Ströme: 8108,3 T. Nitrobenzol + 11208,5 T.H₂ + 589 T. Anilin + 3942, 4 T. H₂O + 1007 T. N₂ (mit Abgasanteil), 210°C; 6, 7 und 8 gemmeinsam: 10810,5 T. H₂ + 6722,6 T. Anilin + 6315,4 T. H₂O + 1007 T. N₂, 400°C; 15: 6022 T. Anilin + 200,6 T. H₂O, 84°C; 16: 107 T. Anilin + 2144 T. H₂O, 60°C; 17: 10810,5 T. H₂ (zurückgeführt 10734 T., ausgeschleust 76,6 T.) + 593,2 T. Anilin (589 T., 4,2 T.) + 3970,5 T. H₂O (3942, 5 T., 28 T.) + 1007 T. N₂ (1000 T., 7 T.), 60°C.

Der Druck lag im Bereich von 5 bis 5,5 bar. Abkühlung nach der Dampferzeugung auf 140°C.

## Patentansprüche

1. Verfahren zur Herstellung von aromatischen Aminen der Formel 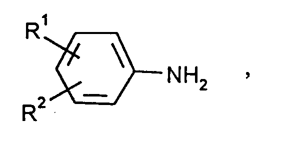 in der
R¹ und R² unabhängig voneinander Wasserstoff, Methyl oder Ethyl bedeuten, wobei R¹ zusätzlich Amino bedeuten kann,
durch Hydrierung von Nitroaromaten der Formel 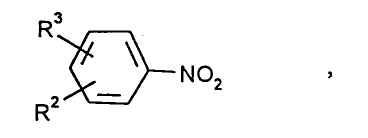 in der
R² und R³ unabhängig voneinander Wasserstoff, Methyl oder Ethyl bedeuten, wobei R³ zusätzlich Nitro bedeuten kann,
mit H₂ an ortsfesten Katalysatoren, dadurch gekennzeichnet, daß bei einem Druck von 1 bis 30 bar, einer Eingangstemperatur des Nitroaromat/H₂-Gemisches von 200 bis 400°C, einem Verhältnis von zwischen 60 und 800 Äquivalenten Wasserstoff pro Nitrogruppenäquivalent nach Homogenisierung der Gasmischung und einer maximalen Katalysatortemperatur von 500°C unter adiabatischen Bedingungen gearbeitet wird.

2. Verfahren nach Anspruch 1, dadurch gekennzeichnet, daß Nitroaromaten der Formel 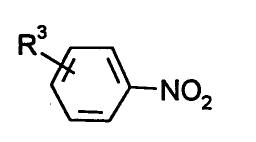 eingesetzt werden, in denen
R³ Wasserstoff, Methyl, Ethyl oder Nitro bedeutet,
und bevorzugt Nitrobenzol eingesetzt wird.

3. Verfahren nach Anspruch 1, dadurch gekennzeichnet, daß die Reaktion in 1-10, bevorzugt 1-5, besonders bevorzugt 1-3 hintereinandergeschalteten Reaktoren durchgeführt wird, von denen jeder Reaktor durch maximal 5, bevorzugt maximal 3, besonders bevorzugt maximal 2 parallel geschaltete Reakoren ersetzt werden kann.

4. Verfahren nach Anspruch 3, dadurch gekennzeichnet, daß nach jedem Reaktor die adiabatisch angestaute Reaktionswärme zur Dampfgewinnung eingesetzt wird.

5. Verfahren nach Anspruch 3, dadurch gekennzeichnet, daß vor jedem Reaktor erneut Nitroaromat dem Reaktionsgemisch zugefügt wird.

6. Verfahren nach Anspruch 1, dadurch gekennzeichnet, daß die Durchdringungstiefe des Katalyatorbettes bei 1 cm - 5 m, bevorzugt 5 cm - 2 m, besonders bevorzugt 10 cm - 1 m, ganz besonders bevorzugt 30 - 60 cm beträgt.

7. Verfahren nach Anspruch 1, dadurch gekennzeichnet, daß ein Palladium auf α-Al₂O₃ enthaltender Katalysator eingesetzt wird, der (a) 1-100 g, bevorzugt 20-60 g, eines oder mehrerer Metalle der Gruppen VIIIa, Ib und IIb des Periodensystems der Elemente (Mendelejew), bevorzugt Pd, (b) 1-100 g, bevorzugt 20-60 g eines oder mehrerer Übergangsmetalle der Gruppen IIb, IVa, Va und VIa, bevorzugt Ti, V, Nb, Ta, Cr, Mo, W und (c) 1-100 g, bevorzugt 10-40 g eines oder mehrerer Hauptgruppenelemente der Gruppen IVb und Vb, bevorzugt Pb, Bi, pro Liter Katalysator auf einem inerten Träger mit einer BET-Oberfläche von weniger als 20 m²/g enthält, wobei (a), (b) und (c) bevorzugt schalenförmig auf dem Träger angeordnet sind und wobei der Katalysator bei einer maximalen Temperatur von 500°C, bevorzugt 480°C, besonders bevorzugt 460°C, ganz besonders bevorzugt 440°C betrieben wird.

8. Verfahren nach Anspruch 7, dadurch gekennzeichnet, daß ein Katalysator eingesetzt wird, der (a) 20-60 g Pd, (b) 20-60 g Ti, V, Nb, Ta, Cr, Mo und/oder W und 10-40 g Pb und/oder Bi pro Liter Katalysator auf α-Al₂O₃ mit einer BET-Oberfläche von weniger als 20 m²/g, bevorzugt von weniger als 10 m²/g,, enthält.

9. Verfahren nach Anspruch 1, dadurch gekennzeichnet, daß ein Palladium auf Kohleträgern enthaltender Katalysator eingesetzt wird, dessen Träger eine BET-Oberfläche von 0,2-10 m²/g aufweist, dessen Pd-Gehalt 0,001-1,5 Gew.-% beträgt, dessen Pd-Menge zu 0-40 Relativ-% durch eines oder mehrere Metalle aus der Grupe von Rh, Ir und Ru ersetzt ist, der zusätzlich einen Gehalt von 0,1-2 Gew.-%, bevorzugt 0,1-1 Gew.-% an einer schwefelhaltigen oder phosphorhaltigen, bevorzugt phosphorhaltigen Verbindung, gerechnet als Schwefel oder Phosphor, haben kann, wobei alle Gewichtsprozente auf das Gesamtgewicht des Katalysators bezogen sind, und der bei einer maximalen Temperatur von 480°C, bevorzugt 460°C, besonders bevorzugt 440°C betrieben wird.

## Claims

1. Process for the production of aromatic amines of the formula 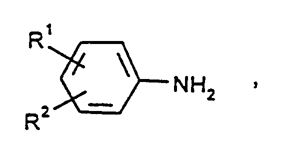 wherein
R¹ and R² independently of one another denote hydrogen, methyl or ethyl, wherein R¹ can also denote amino,
by hydrogenation of nitroaromatics of the formula 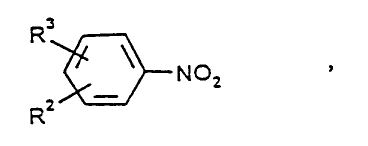 wherein
R² and R³ independently of one another denote hydrogen, methyl or ethyl, wherein R³ can also denote amino,
using H₂ on fixed catalysts, characterised in that the process is carried out under adiabatic conditions at a pressure of from 1 to 30 bar, at an inlet temperature of the nitroaromatic/H₂ mixture of from 200 to 400°C, with a proportion of between 60 and 800 equivalents of hydrogen per nitro-group equivalent after homogenisation of the gas mixture and at a maximum catalyst temperature of 500°C.

2. Process according to claim 1, characterised in that nitroaromatics of the formula 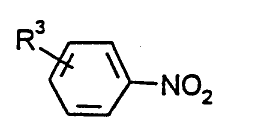 are used, wherein
R³ denotes hydrogen, methyl, ethyl or nitro,
and preferably nitrobenzene is used.

3. Process according to claim 1, characterised in that the reaction is carried out in from 1 to 10, preferably 1 to 5, particularly preferably 1 to 3 reactors connected in series and each of the said reactors can be substituted by at most 5, preferably at most 3, particularly preferably at most 2 reactors connected in parallel.

4. Process according to claim 3, characterised in that after each reactor the adiabatically accumulated heat of reaction is used for the recovery of vapour.

5. Process according to claim 3, characterised in that, upstrearn from each reactor, nitroaromatic is again fed to the reaction mixture.

6. Process according to claim 1, characterised in that the penetrable depth of the catalyst bed is from 1 cm to 5 m, preferably from 5 cm to 2 m, particularly preferably from 10 cm to 1 m, and most preferably from 30 cm to 60 cm.

7. Process according to claim 1, characterised in that a catalyst containing palladium on α-Al₂O₃ is used, which catalyst contains (a) from 1 to 100 g, preferably from 20 to 60 g, of one or more metals of the groups VIIIa, Ib and IIb of the periodic table of elements (Mendeleev), preferably Pd, (b) from 1 to 100 g, preferably from 20 to 60 g of one or more transition metals of the groups IIb, IVa, Va and VIa, preferably Ti, V, Nb, Ta, Cr, Mo, W and (c) from 1 to 100 g, preferably from 10 to 40 g of one or more main group elements of the groups IVb and Vb, preferably Pb, Bi, per litre of catalyst on an inert support having a BET surface area of less than 20 m²/g, with (a), (b) and (c) preferably being arranged in the form of a shell on the support and with the catalyst being operated at a maximum temperature of 500°C, preferably 480°C, particularly preferably 460°C and most preferably 440°C.

8. Process according to claim 7, characterised in that a catalyst is used which contains (a) from 20 to 60 g of Pd, (b) from 20 to 60 g of Ti, V, Nb, Ta, Cr, Mo and/or W and from 10 to 40 g of Pb and/or Bi per litre of catalyst on α-Al₂O₃ having a BET surface area of Iess than 20 m²/g, preferably of less than 10 m²/g.

9. Process according to claim 1, characterised in that a catalyst is used which contains palladium on carbon supports, the said catalyst support material has a BET surface area of from 0.2 to 10 m²/g, the catalyst has a Pd content of from 0.001 to 1.5 wt.%, the said quantity of Pd can be replaced by from 0 to 40 percent, relative to Pd, of one or more metals of the group comprising Rh, Ir and Ru, the catalyst can possess in addition a content of from 0.1 to 2 wt.%, preferably from 0.1 to 1 wt.%, of a sulfur-containing or phosphorus-containing, preferably phosphorus-containing compound, calculated as sulfur or phosphorus, with all the percentages by weight referring to the total weight of the catalyst, and is operated at a maximum temperature of 480°C, particularly preferably 460°C and most preferably 440°C.

## Revendications

1. Procédé de préparation d'amines aromatiques de formule : 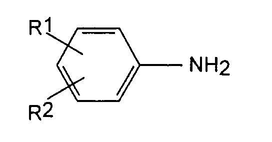 dans laquelle :
R¹ et R² représentent indépendamment l'un de l'autre, hydrogène, méthyle ou éthyle, où R¹ peut représenter, en outre, amino,
par hydrogénation de nitroaromatiques de formule : 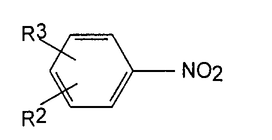 dans laquelle :
R² et R³ représentent indépendamment l'un de l'autre, hydrogène, méthyle ou éthyle, où R³ peut représenter, en outre, nitro,
avec H₂, sur un catalyseur fixe, caractérisé en ce que l'on travaille, pour une pression allant de 1 à 30 bar, à une température d'entrée du mélange nitroaromatique/H₂ allant de 200 à 400°C, à un rapport allant de 60 à 800 équivalents d'hydrogène par équivalent de radical nitro après homogénéisation du mélange gazeux, et à une température maximale du catalyseur de 500°C, dans des conditions adiabatiques.

2. Procédé suivant la revendication 1, caractérisé en ce que l'on met en oeuvre des nitroaromatiques de formule : 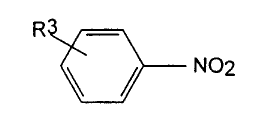 dans laquelle
R³ représente hydrogène₁ méthyle, éthyle ou nitro,
et de préférence, on met en oeuvre le nitrobenzène.

3. Procédé suivant la revendication 1, caractérisé en ce que la réaction est réalisée dans 1 à 10, de préférence 1 à 5, de manière particulièrement préférée de 1 à 3 réacteurs disposés en série, dont chacun des réacteurs peut être remplacé par au maximum 5, de préférence au maximum 3, de manière particulièrement préférée au maximum 2 réacteurs disposés en parallèle.

4. Procédé suivant la revendication 3, caractérisé en ce que après chaque réacteur, la chaleur de réaction adiabatique est mise en oeuvre pour produire de la vapeur.

5. Procédé suivant la revendication 3, caractérisé en ce que avant chaque réacteur, on ajoute au mélange réactionnel, du nouveau nitroaromatique.

6. Procédé suivant la revendication 1, caractérisé en ce que la profondeur de pénétration du lit de catalyseur se situe dans l'intervalle allant de 1 cm à 5 cm, de préférence de 5 cm à 2 m, de manière particulièrement préférée de 10 cm à 1 m, de manière tout particulièrement préférée de 30 cm à 60 cm.

7. Procédé suivant la revendication 1, caractérisé en ce que l'on met en oeuvre un catalyseur contenant du palladium sur α-Al₂O₃, qui contient (a) 1-100 g, de préférence 20-60 g d'un ou plusieurs métaux des groupes VIIIa, Ib et IIb du système périodique des éléments (Mendéléev), de préférence Pd ; (b) 1-100 g, de préférence 20-60 g d'un ou plusieurs métaux de transition des groupes IIb, IVa, Va et VIa, de préférence Ti, V, Nb₁ Ta, Cr, Mo, W et
(c) 1-100 g, de préférence 10-40 g d'un ou plusieurs éléments des groupes principaux des groupes IVb et Vb, de préférence Pb, Bi, par litre de catalyseur sur un support inerte ayant une surface BET inférieure à 20 m²/g, où (a), (b) et (c) sont disposés de préférence sur le support, sous forme d'une cuvette et où le catalyseur est mis en oeuvre à une température maximale de 500°C, de préférence de 480°C, de manière particulièrement préférée de 460°C, de manière tout particulièrement préférée de 440°C.

8. Procédé suivant la revendication 7, caractérisé en ce que l'on met en oeuvre un catalyseur qui contient (a) 20-60 g de Pd, (b) 20-60 g de Ti, V, Nb, Ta, Cr, Mo et/ou W, et (c) 10-40 g de Pb et/ou Bi par litre de catalyseur, sur de l'α-Al₂O₃ ayant une surface BET inférieure à 20 m²/g, de préférence inférieure à 10 m²/g.

9. Procédé suivant la revendication 1, caractérisé en ce que l'on met en oeuvre un catalyseur contenant du palladium sur un support au charbon, dont le support présente une surface BET allant de 0,2 à 10 m²/g, dont la teneur en Pd se situe dans l'intervalle allant de 0,001 à 1,5% en poids, dont la quantité de Pd est remplacée à 0-40% relatifs par un ou plusieurs métaux parmi le groupe des Rh, Ir et Ru, qui peut avoir en outre, une teneur allant de 0,1 à 2% en poids, de préférence de 0,1 à 1% en poids en un composé soufré ou phosphoré, de préférence phosphoré, calculé comme soufre ou phosphore, où les pourcentages en poids se rapportent au poids total du catalyseur, et qui fonctionne à une température maximale de 480°C, de préférence 460°C, de manière particulièrement préférée de 440°C.
